# EUROPEAN PATENT APPLICATION

(11) **EP 2 745 844 A1**
(43) Date of publication of application: **25.06.2014**
(21) Application number: 12823903.5
(22) Date of filing: 15.08.2012
(51) Int. Cl.: A61K 39/00, A61P 31/18

(54) **MOLECULAR MIMIC MUCOSAL AIDS VACCINE**

(30) Priority: 15.08.2011 JP 2011177385
(71) Applicant: National University Corporation Kumamoto University, Kumamoto-shi Kumamoto 860-8555 (JP); LSIP, LLC, Chiyoda-ku Tokyo 100-0005 (JP)
(72) Inventor: MISUMI, Shogo, Kumamoto-shi Kumamoto 862-0973 (JP); SHOJI, Shozo, Kumamoto-shi Kumamoto 862-0973 (JP); TAKAMUNE, Nobutoki, Kumamoto-shi Kumamoto 862-0973 (JP)
(74) Representative: Cabinet Plasseraud
(86) International application number: PCT/JP2012/070724
(87) International publication number: WO 2013/024859

(57) **Abstract**

The present invention addresses the problem of providing an AIDS vaccine whereby mucosal immunity can be induced, the immune system can be defended against breakdown by HIV infection, HIV can be prevented from escaping the immune system by being highly mutable, and rapid HIV infection can be prevented. According to the present invention, provided is an AIDS vaccine comprising a hub antigen and a complex of N²,N⁶-bis [N²,N⁶-bis(3,4,5-trihydroxybenzoyl)-L-lysyl]-N-(2-aminoethyl)-L-lys-inamide (TGDK) and fetuin.

## Description

### TECHNICAL FIELD

The present invention relates to a molecular mimic mucosal AIDS vaccine for mucosal immunity.

### BACKGROUND ART

The vaccine started with the smallpox shot by Edward Jenner in 1796 and after its conceptualization by Louis Pasteur, the vaccine has become an essential tool for biological defense. Although the molecular mechanism of biological defense by vaccination has been partly unraveled, much remains yet to be known. There is some misunderstanding about side reactions of vaccines and, in the past, inappropriate ways of their administration (such as needle sharing) have caused the problem of increased incidence of new infections (such as type C hepatitis.). HIV/AIDS vaccines have been mostly developed under the initiative of the United States of America but to date no substantial success has been gained.

Patent Document 1 discloses an intestinal immunity activator which comprises a compound represented by formula 1: TGDK-CH₂-CH₂-NH-R (wherein TGDK-CH₂-CH₂-NH- represents 2-[N-α, N-ε-bis(N-α, N-ε-digalloyllysinyl)lylsinyl]aminoethylamino group; R represents a moiety selected from the group consisting of a hydrogen atom, a group having an active ester group via a peptide bond, a group binding to a SH group via a peptide bond, a peptide, a protein, a lipid or a sugar that are bound via a peptide bond, or a peptide, a protein, a lipid or a sugar that are bound via a Schiff base). Patent Document 1 further discloses that the above-defined TGDK recognizes M cells.

### CITATION LIST

### PATENT LITERATURE

Patent Document 1: International Publication WO 2007/052641 A1 (International Application Number PCT/JP 2006/321720)

### SUMMARY OF INVENTION

### TECHNICAL PROBLEM

The problem to be solved by the present invention is to provide an AIDS vaccine that is capable of inducing mucosal immunity, defending against breakdown of the immune system due to HIV infection, preventing HIV from escaping the immune system on account of its high mutability, and preventing rapid infection of HIV.

### SOLUTION TO PROBLEM

To solve the above-described problem, the present inventors made intensive studies focusing on a human protein structurally homologous to HIV Env gp120 envelope glycoprotein and commercially available under the trade name Fetuin (product of Sigma). Presumably, HIV may change the structure of gp120 envelope protein into that of Fetuin (a molecular mimicry protein) to escape the barrier of the human immune system. Making use of this protein, the present inventors synthesized a covalent complex which is prepared by conjugating a Hub-antigen, N²,N⁶-bis[N²,N⁶-bis(3,4,5-trihydroxybenzoyl)-L-lysyl]-N-(2-aminoethyl)-L-lysine amide (TGDK), and Fetuin via covalent bond; as a result, the inventors could successfully developed a molecular mimicry mucosal vaccine against HIV/AIDS.

Thus, according to the present invention, there is provided an AIDS vaccine comprising a covalent complex of a Hub-antigen, N²,N⁶-bis[N²,N⁶-bis(3,4,5-trihydroxybenzoyl)-L-lysyl]-N-(2-aminoethyl)-L-lysine amide (TGDK), and Fetuin.

Preferably, the AIDS vaccine of the present invention is administered subcutaneously and/or orally and nasally.

### ADVANTAGEOUS EFFECTS OF INVENTION

The AIDS vaccine of the present invention has the advantages capable of inducing mucosal immunity, defending against breakdown of the immune system due to HIV infection, preventing HIV from escaping the immune system on account of its high mutability, and prevent rapid infection of HIV.

### BRIEF DESCRIPTION OF DRAWINGS

FIG. 1, A and B, shows how X-protein (Fetuin) can be identified.
FIG. 2 shows the sequence of X-protein.
FIG. 3 shows the alignment between the amino acid sequences of SIV gp120 and X-protein.
FIG. 4 structurally compares X-protein with an HIV Env trimer, in which moieties in a conformation of gp120 that are homologous to X-protein (Fetuin) are indicated in yellow.
FIG. 5 shows UPA-like structures in the sequence of X-protein (Fetuin).
FIG. 6 shows the results of measurement of anti-gp140 antibody titers.

### DESCRIPTION OF EMBODIMENTS

The present invention will hereinafter be described in greater details.

The present invention relates to an AIDS vaccine comprising a covalent complex of a Hub-antigen, N²,N⁶-bis[N²,N⁶-bis(3,4,5-trihydroxybenzoyl)-L-lysyl]-N-(2-aminoethyl)-L-lysine amide (TGDK), and Fetuin.

The Hub-antigen to be used in the present invention is a glycerol polyethylene glycol polymer having a large number of primary amines on its surface that is produced by the following method: the active ester scaffold in HGEO-200NP (product of YUKA SANGGYO CO., LTD.) serving as the core structure is reacted with a small excess of HGEO-200PA (product of YUKA SANGGYO CO., LTD.), whereupon the molecule of HGEO-200PA forms a hub (a center from which others radiate in a wheel-like figure).

The N²,N⁶-bis[N²,N⁶-bis(3,4,5-trihydroxybenzoyl)-L-lysyl]-N-(2-aminoethyl)-L-lysine amide (TGDK) is a known compound and the method of its synthesis is disclosed in the above-mentioned Patent Document 1 (International Publication WO 2007/052641 A1), for example.

Fetuin is a protein synthesized in the liver from which it is secreted into blood. Fetuin is a kind of binding proteins that mediate transport and utilization of a wide range of transporter proteins in the blood circulation. Human Fetuin is synonymous with α2-HS-glycoprotein (AHSG), α2-HS, A2HS, AHS, HSGA, and Fetuin-A.

The method of synthesizing the covalent complex of the Hub-antigen, N²,N⁶-bis[N²,N⁶-bis(3,4,5-trihydroxybenzoyl)-L-lysyl]-N-(2-aminoethyl)-L-lysine amide (TGDK), and Fetuin is not particularly limited. An exemplary method of synthesis involves the procedure to be described later in the Examples section but this is not the sole method that can be employed. According to the method described later in the Examples section, activated polyethylene glycol (PNP4(10)) is first reacted with TGDK to synthesize an active ester of TGDK (i.e., PNP-TGDK). Then, this PNP-TGDK is reacted with the Hub-antigen to yield Hub-TGDK-PNP. Finally, this Hub-TGDK-PNP is reacted with Fetuin to thereby synthesize a covalent complex of Hub-TGDK-Fetuin.

The complex Hub-TGDK-Fetuin in the AIDS vaccine of the present invention may be used as it is or it may be formulated as a suitable form of preparation together with a pharmaceutically acceptable carrier. Exemplary carriers that may be used include excipients, binders, disintegrants, lubricants, and so on. The form of the preparation is not particularly limited and it may assume any forms such as liquids, tablets, pills, capsules, powders, granules, and syrups.

Examples of excipients that can be used include sugars such as lactose, sucrose, and glucose; starches such as potato starch and wheat starch; celluloses such as microcrystalline cellulose; inorganic salts such as anhydrous calcium hydrogen phosphate and calcium carbonate; polyethylene glycols and so on. Examples of binders that can be used include microcrystalline cellulose, pullulan, gum arabic, sodium alginate, and polyvinylpyrrolidone, as well as multifunctional polyethylene glycols. Examples of disintegrants that can be used include carboxymethyl cellulose, carboxymethyl cellulose calcium, hydroxypropyl cellulose, hydroxypropyl starch, and sodium alginate, as well as polyethylene glycols. Examples of lubricants that can be used include magnesium stearate, talc, and hardened oils. Examples of nanoparticle formers include cholesterol pullulan and multifunctional polyethylene glycols (MOF CORPORATION).

The route by which the AIDS vaccine of the present invention is to be administered is not particularly limited and it may be oral or parenteral route (for example, subcutaneous, rectal, intramuscular, nasal, or intravenous); a preferred route of administration is subcutaneous and/or oral and nasal route.

The amount of the active ingredient to be contained in the AIDS vaccine of the present invention (i.e., the covalent complex represented by Hub-TGDK-Fetuin) can be appropriately determined depending on the age, body weight, symptoms, etc. of the subject of administration or the patient; it is typically adjusted to range from 1 µg to 1000 mg/kg/dose, preferably from 10 µg to 100 mg/kg/dose.

The AIDS vaccine of the present invention may be administered together with an adjuvant. Any substance can be used as the adjuvant if it is capable of augmenting immune responses by being administered prior to the vaccine (antigen). Examples include not only antigenic adjuvants such as pasteurized microorganisms but also non-antigenic adjuvants such as alum (aluminum hydroxide potassium salt) and mineral oils. Jules Freund discovered in 1947 that when an aqueous solution of an antigen was mixed with an equal volume of oil (85% mineral oil and 15% surfactant) and injected in the form of an emulsion, an antibody was produced in an increased amount. Today, this is called incomplete Freund's adjuvant (IFA) and may be supplemented with whole cells of *M. tuberculosis* to form complete Freund's adjuvant (CFA). Other substances that exhibit the adjuvant effect are mineral acid salts such as aluminum hydroxide and aluminum phosphate. In addition, endotoxins, particularly lipopolysaccharides from Gram-negative bacteria, can markedly promote antibody production, with their active ingredient being present in lipid A. In the present invention, any one of the substances mentioned above can be used as adjuvants. The route of administration of the adjuvants is not particularly limited and may be oral or parenteral.

The distinctive features of the AIDS vaccine of the present invention are explained below.

### (1) Induction of mucosal immunity

The TGDK (tetragalloyl D-lysine) derivative was discovered as a competitive inhibitor of UAE-1 (C-type lectin) and the present inventors successfully synthesized TGDK in both a solid and a liquid phase as an M cell targeting molecule essential to mucosal vaccines (Misumi et al., J. Immunol. 182, 6061-6070 (2009)). To be more specific, TGDK targeting at M cells is transcytosed to the lamina propria and binds to both NK-cells (natural killer cells), expressed by CD161, and NKT-cells (natural killer T-cells), mediating between the natural immune system and an acquired immune system, whereby the whole immune system is activated. In addition, the vaccine having formed a covalent bond with TGDK can induce systemic immunity and, with a small time lag, mucosal immunity even if it is injected subcutaneously, regardless of the type of the administered antigen.

### (2) Defense against breakdown of the immune system due to HIV infection

In the process of infecting cells that play central roles in anti-HIV immunity, HIV makes use of CCR5 or CXCR4 (chemokine receptors) as a co-receptor. The present inventors focused on a specific conformational site of this co-receptor. The extracellular loop 2 (ECL-2) of either co-receptor forms a disulfide bond with ECL-1 and the resulting arch which is composed of 11 amino acid residues displays a specific conformation, called a UPA structure. When the disulfide bond forming this UPA structure is reductively cleaved and the arch breaks, HIV is no longer infective. In the molecules of the co-receptors to be utilized by HIV, the UPA structure is the most important moiety. As a preliminary step to induce a specific antibody against this UPA structure, a cyclic peptide mimicking it was synthesized to develop a cyclic antigenic vaccine. A mucosal immune tissue of a monkey immunized with this vaccine prevented breakdown of the immune system due to the immune tissue's destruction resulting from HIV infection, whereby the viral infection could be significantly suppressed (Misumi et al., J. Virol. 75, 11614-11620 (2001); Misumi et al., J. Biol. Chem. 278, 32335-32343 (2003)). According to the present invention, defense against breakdown of the immune system can be achieved by inducing a specific antibody against the special conformation of either co-receptor of HIV.

### (3) Prevention of easily mutagenic HIV-escape from the immune system

HIV forms a trimer of its own envelope glycoprotein (spike protein) before it adsorbs to a cell and invades it. The mechanism by which HIV escapes the immune system on account of its high mutability may be explained as follows: a single base mutation of a virus gene leads to a mutation of a single amino acid residue, which is reflected in a "fluctuation" of the conformation of the viral envelope glycoprotein on account of the breaking of hydrogen bonds; a high-affinity neutralizing antibody evolved and induced *in vivo* in order to be specifically compatible can no longer recognize the "fluctuation" resulting that it does not react with the mutated antigen and fails to detoxify it. To prevent the HIV from escaping the immune system, the present inventors applied the following procedure in genetic chemistry as a means of facilitating the trimer formation: mutations in two amino acid residues (K514E and K524E) were inserted into the Ecto-domain gene of HIV Env protein to make it resistant to processing with an enzyme, thereby facilitating the trimer formation; there was also inserted a site capable of binding of the native protein via nickel; however, the sites subject to sugar chain modifications were left intact. A structural molecule that would reflect the conformational "fluctuation" due to a viral mutation was constructed in the following way: the conformational "fluctuation" due to a mutation of a virus was taken as a "fluctuation" of the molecular structure due to the breaking of hydrogen bonds in an aqueous solution of the polar solvent dimethylformamide; based on this assumption, the viral envelope glycoprotein was treated in an aqueous solution of the polar solvent dimethylformamide in a concentration-dependent manner (0 to 50% concentration in water) and the structure of the concurrently occurring "fluctuation" was covalently bonded to the activated Hub (radiant glycerol-polyethylene glycol)-TGDK antigen to prepare the desired molecular structure. The antibody induced by this antigen was independent of the viral strain and could neutralize a variety of viruses.

### (4) Prevention of rapid infection of HIV

When immunized with a vaccine (pseudo-pathogenic antigen), humans will generally acquire immunity in 2-3 weeks (primary immune response). As the primary immune response subsides and memory immunity is formed, a human will immediately react to an invading, genuine pathogenic antigen and immunity is reactivated within a very short period of 12-24 hours (secondary immune response); as a result, the immune system wins the battle with the pathogenic antigen which is expelled, bringing the subject back to a healthy state. In the case of HIV, however, the virus sneaks into a gene of an immune cell during the 12-24 hour period of the secondary immune response and is incorporated into the gene to establish infection. Once incorporated into the gene, the HIV's gene cannot be expelled. In the process of infecting the immune cell, HIV proliferates as it destroys the cell and eventually the immune system loses the battle and AIDS is manifested in the human. This is why HIV must never be permitted to invade. To prevent rapid infection of HIV, a phenomenon called "induction of cross-immunity" is invoked in the mucous membrane, where initial infection takes place, to ensure that neutralizing antigens or antigen-binding antibodies are made available at all times; if this is achieved, the invading virus can be expelled with the aid of the natural immune system and the complement system; in addition, some design for maintaining killer T-cells need be worked out. In the present invention, the Hub-TGDK antigen was bound to the native HIV Env glycoprotein antigen and an escort antigen invoking induction of cross immunity was covalently bonded to prepare a multivalent covalent-bound antigen. Using this multivalent covalent-bound antigen, basic immunization was performed and, thereafter, antigens having moieties homologous to multivalent antigens other than the vaccine's native HIV Env glycoprotein antigen from a viewpoint of structural biology were used under memory immunity to construct on the mucous membrane a cross immunity framework capable of inducing antigens reacting with the HIV Env glycoprotein. According to the present invention, an immunity framework capable of invoking induction of cross immunity could be constructed in a monkey and the biological component Fetuin was discovered as an antigen capable of inducing cross immunity. By performing basic immunization with the viral glycoprotein antigen, the present inventors successfully developed a cross immunity inducing vaccine capable of producing an anti-HIV Env antibody with Fetuin instead of the viral glycoprotein antigen. As a result, the present inventors established a novel method of vaccination that would completely preclude HIV invasion into a local site in the mucous membrane.

The present invention will be illustrated more specifically by reference to the following Examples, however, the present invention is by no means limited by these Examples.

### EXAMPLES

Example 1: Molecular Mimetic Mucous Vaccine for Biological Defense / Identification of Molecular Mimicry Moiety in Structural Biology Against HIV Envelope Glycoprotein (HIV Env gp120)

### Example 1-1: Identification of Molecular Mimicry Moiety

The present inventors previously created TGDK targeting to mucosal M cells (Misumi et al., J. Immunol. 182, 6061-6070 (2009)) and further created cyclic peptide antigens inducing antibodies against special conformations (undecapeptidyl arch; UPA) of the HIV co-receptors CCR5 and CXCR4 (Misumi et al., J. Virol. 75, 11614-11620 (2001) and Misumi et al., J. Biol. Chem. 278, 32335-32343 (2003)). The induced antibodies were capable of preventing breakdown of the immune system by HIV. In addition, to cope with the high mutability of HIV, the present inventors made attempts at obtaining antibodies having a wide range of binding specificity that would respond in spite of HIV mutation, in other words, in spite of slight changes in the antigen; to meet this end, the present inventors created a monomer and a trimer of the antigen to be used and predicted a conformational "fluctuation" due to HIV's mutation on the basis of the breaking of hydrogen bonds, and coped with the mutation-induced escape of HIV from the immune system. As a measure for facilitating the formation of a trimer of SIVmac239 Env protein, the two amino acid residues in the Ecto-domain at the C-terminal side of gp130 protein were altered to make this protein less susceptible to the action of an in vivo protease. A gene capable of producing a Ni-binding peptide was inserted at the C-terminus while the other portions were allowed to maintain a structure subject to sugar chain modifications similar to the native Env protein.

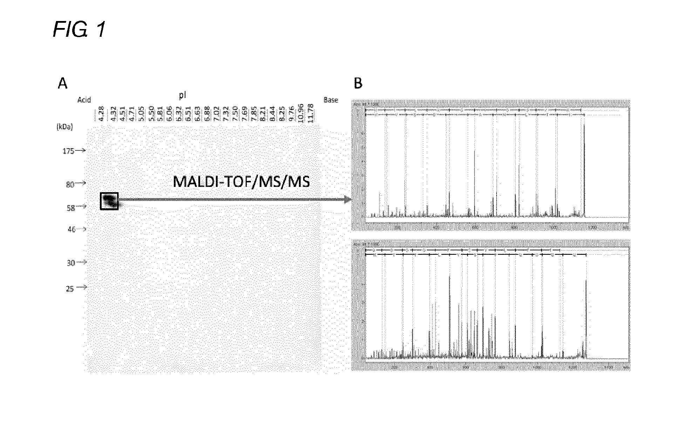

The resulting modified gene of SIVmac239 Env was transferred into simian Vero cells and a monomer and a trimer of SIVmac239 Env gp140 were constructed. In the study on designing an HIV/AIDS mucosal immune vaccine (Misumi et al., J. Immunol. 182, 6061-6070 (2009)), the present inventors discovered the induction of cross immunity in an immunization experiment using monkeys as follows. A vaccine was created by forming covalent bond with the Hub-antigen (glycerol-polyethylene glycol) to TGDK (agent targeting mucosal M cells), UPA (undecapeptidyl arch of CCR5; antigen inducing antibody against immunity destruction), CpGODN (B cell activator), and SIVmac239 Env (gp140 protein) as well as X-protein (Fetuin); after basic immunization of monkeys with this vacine, there occurred immune response which then subsided and when the anti-gp140 antibody titer decreased (after about one year and a half), the monkeys were immunized with a vaccine free of the gp140 antigen, whereupon the anti-gp140 antibody was induced despite the absence of the gp140 antigen; this anti-serum neutralized SIV and HIV, as well as members of their subspecies group. The present inventors discovered an antigenic molecule that would elicit the induction of cross immunity of interest and identified it to be Fetuin as the result of analyses by electrophoresis and MALDI-TOF/MS/MS (Figs. 1A and 1B.)

The same monkeys were subcutaneously injected with Fetuin alone and the production of anti-gp140 antibodies was confirmed. This protein is a ubiquitous protein which had an identical part, a homologous part, and a homologous moiety with respect to the monomer and trimer of SIVmac239 Env gp140 from a viewpoint of structural biology; being an extremely unique glycoprotein molecule, this antigen also had the same features with respect to HIV Env as well as SIV Env. The molecule was named an HIV molecular mimicry protein.

### Example 1-2: Characterization of HIV Molecular Mimicry Protein

### (1) Amino acid sequence and chemically modified sites

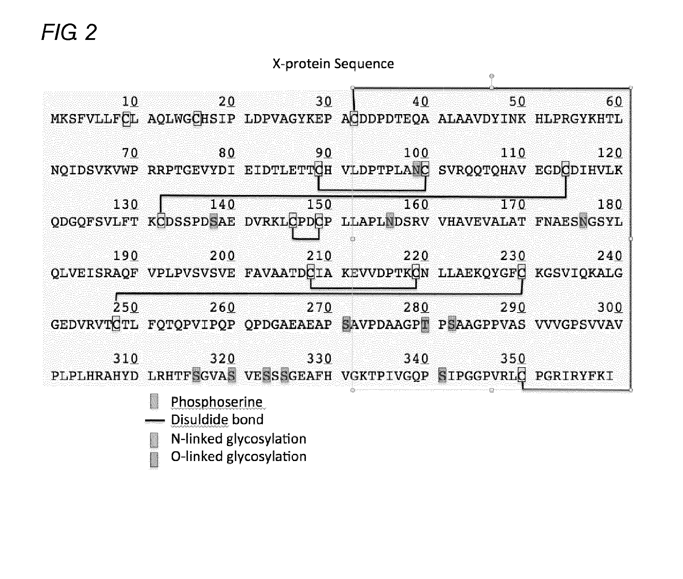

The amino acid sequence of the HIV molecular mimicry protein is depicted in Fig. 2. The HIV molecular mimicry protein has N-linked glycosylation sites, O-linked glycosylation sites, phosphorylation sites, six S-S bridges, and two free SH groups.

### (2) Comparison between the amino acid sequences of HIV molecular mimicry protein and HIV Env

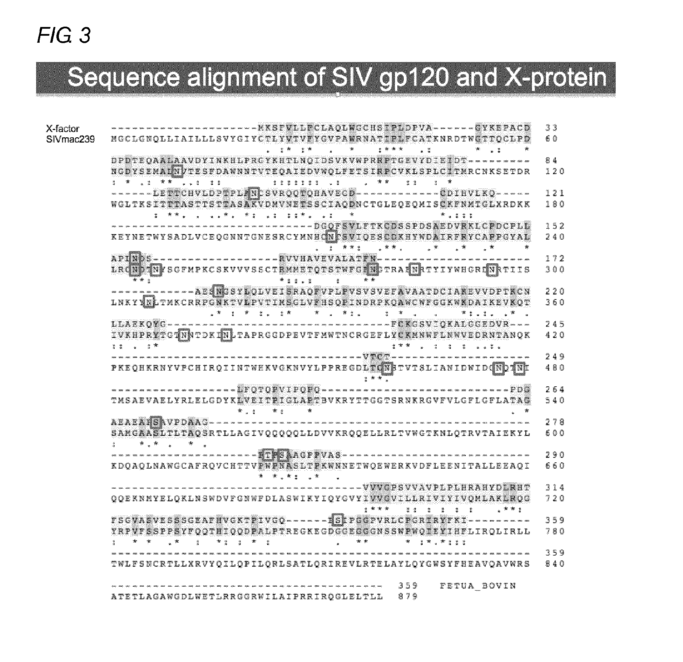

The amino acid sequences of HIV molecular mimicry protein and HIV Env are depicted in Fig. 3, in which the moieties likely to be subject to an addition of sugar chains are enclosed with a square.

### (3) Comparison between the conformation of HIV molecular mimicry protein and the structure of HIV Env trimer

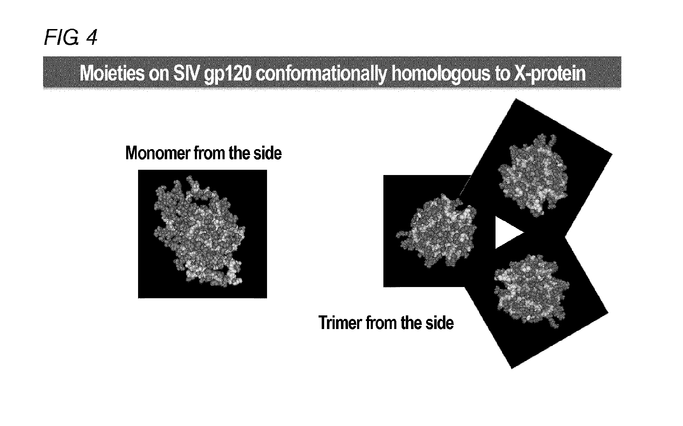

The conformation of the HIV molecular mimicry protein was compared with the structure of HIV Env trimer and the results are shown in Fig. 4, in which conformationally homologous moieties are indicated in yellow. As shown in Fig. 4, homologous moieties were found to exist between the conformation of the HIV molecular mimicry protein and the structure of HIV Env trimer.

### (4) Characterization of the conformation of HIV molecular mimicry protein due to S-S bridges

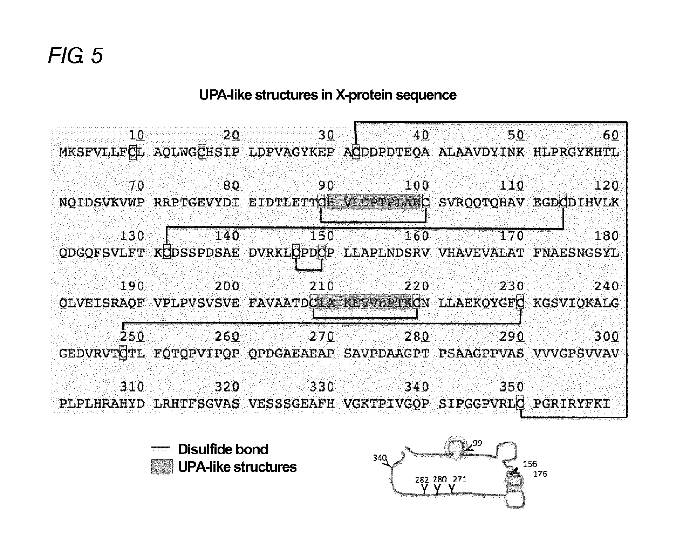

The sites of S-S bridges in the HIV molecular mimicry protein are shown in Fig. 5. Two of the six S-S bridges formed an arch spanning 10 or 11 amino acid residues. The arch structures at these two sites were found to be homologous to the undecapeptidyl arch (UPA) of second extracellular loop (ECL-2) in the HIV-1 co-receptor CCR5 or CXCR4 (Misumi et al., J. Virol. 75, 11614-11620 (2001) and Misumi et al., J. Biol. Chem. 278, 32335-32343 (2003)).

### (5) Summary of the characterization of HIV molecular mimicry protein

The HIV molecular mimicry protein was found to be a biological component having an extremely unique conformation in that it was quite homologous to the structure of the HIV Env trimer and that it also had the UPA structure of the HIV-1 co-receptor CCR5 or CXCR4.

### Example 2: Preparation of HIV/AIDS Molecular Mimicry Mucosal Vaccine (MMMV)

### Example 2-1: Chemical Structural Formula of TGDK

Chemical name of TGDK: N²,N⁶-bis[N²,N⁶-bis(3,4,5-trihydroxybenzoyl)-L-lysyl]-N-2-aminoethyl)-L-lysine amide (TGDK)

### Gal: Galloyl group (trihydroxybenzoyl group)

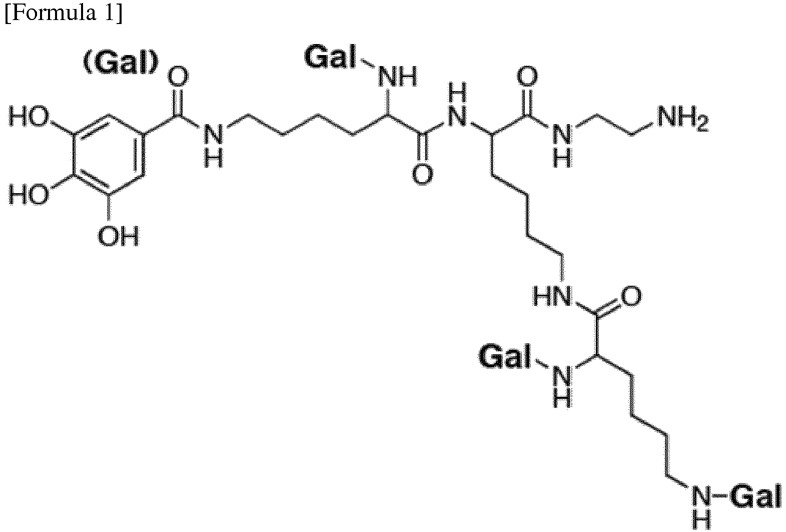

### Example 2-2: Purification of TGDK (homogenous on MS chart; salt was removed by this procedure)

TGDK was metered in an amount of 12 µmole and after adding DMF (dehydrated) (500 µl) and ether (dehydrated) (1500 µl), the mixture was centrifuged at 15000 rpm for a minute. To the precipitate, 100% NMM (dehydrated) (600 µl) was added and the mixture was sonicated for 1-2 seconds and then centrifuged at 15000 rpm for a minute. To the precipitate, 100% TFA (100 µl) was added and after adding ether (dehydrated) (900 µl) to the supernatant, the mixture was centrifuged at 15000 rpm for a minute. To the precipitate, DMF (dehydrated) (600 µl) and 100% NMM (dehydrated) (100-300 µl) were added in that order. The pH of the resulting solution was confirmed as follows: to 1 µl of the solution, 4 µ of water was added and pH measurement was done (pH 8-9). This gave a solution of purified TGDK in DMF (TGDK 12 µmole; weakly alkaline).

### Example 2-3: Synthetic schemes of Hub-HGDK-Fetuin

In the following descriptions, activated polyethylene glycol (activated PEG, PTE-100NP; product of YUKA SANGYO CO., LTD.) is abbreviated as PNP4 (10), in which PNP refers to an activated ester.

### (1) Synthesis of PNP-TGDK

To PNP4(10) (PTE-100NP, 18 µmole, 180 mg (total PNP, 18 x 4 x 0.9 = 64.8 µmole)) (180 mg), DMF (dehydrated) (900 µl) and purified TGDK (12 mole) (300-600 µl) were added in that order and the mixture was subjected to reaction at room temperature for 3 hours. The pH of the reaction mixture was measured as follows: to 1 µl of the reaction mixture, 4 µl of water was added and pH was confirmed (pH 8-9). This gave a solution of PNP-TGDK (1.5 ml, residual PNP: 18 x 3.6 = 64.8 - 12 = 52.8 µmole) (193 mg).

### (2) Synthesis of Hub-TGDK-Fetuin

To the Hub-antigen (A8-49, 1 µmole, 168 mg, NH₂ 49 µmole), DMF (dehydrated) (22 ml) and PNP-TGDK (12 µmole) (1.5 ml) were added in that order (residual PNP: 52.8-49 = 3.8 µmole). For pH measurement, 4 µl of water was added to 1 µl of the reaction mixture and pH was confirmed as described above (pH 7.6-8.4) (193 mg). The mixture was then left to stand for 12 hours at room temperature to effect reaction and after the end of the reaction, pH was measured and confirmed by the procedure described above (pH 7.3-8). This gave Hub-TGDK-PNP (residual amount: 3.8 µmole) (23 ml).

To this mixture, Fetuin (50 mg, 0.9 µmole) / PBS(-) (23 ml) (pH 7.4-7.8) was rapidly added dropwise (for a few minutes). The resulting mixture was stirred at room temperature overnight and dialyzed (Mw cutoff, 3500) under the following conditions.
1. Against PBS(-), 2 L x three times, 24 hours
2. Against purified water, 2 L x three times, 24 hours
3. Against secondary water, 2 L x once, 12 hours.

The dialyzates were lyophilized to give lyophilized powder (about 130 mg of a Hub-TGDK-Fetuin powder).

### Example 2-4: Sterilization

Sterilization with 70% EtOH produced visible gelation, so in the usual manner, the gel was stirred to homogeneity under sterilization.

### Example 3: Administration to Monkey of the HIV/AIDS Molecular Mimicry Mucous Vaccine

### (1) Experimental materials and method:

Six female cynomolgus monkeys (3 for the control group and 3 for the immunization) weighing about 3.5-4.5 kg were used in the experiment. The three monkeys of the immunization group were subcutaneously injected at both groins with 5 mg of the vaccine (Hub-TGDK-Fetuin; effective amount of Fetuin, ca. 1 mg) per monkey in 1 ml of PBS(-). Serum samples were prepared every two weeks.

### (2) Measurement of antibody titer

The anti-gp140 antibody titer was measured by ELISA in accordance with the usual procedure.

### (3) Results and discussion

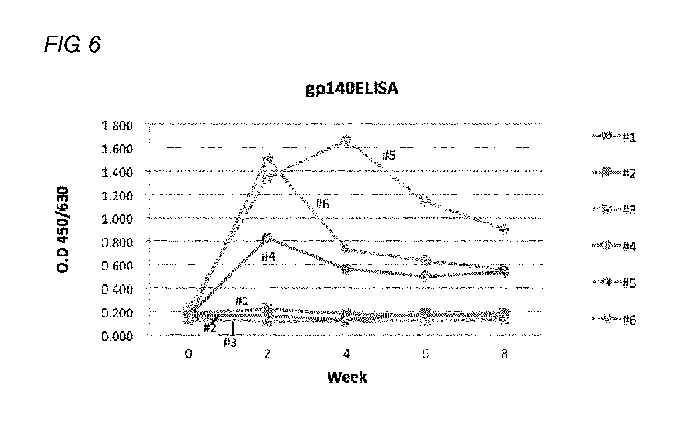

The results of the antibody titer measurement are shown in Fig. 6.

In 2 weeks after the subcutaneous injection, the anti-gp140 antibody titer was found to elevate in the three vaccinated animals and in 4 weeks, the titer reached a maximum; in 8 weeks, it decreased to about 40-50% levels as compared to the maximum value, which have been maintained up until the present time. The anti-sera obtained at 8 weeks could prevent the infection with HIV-1 and SIVmac239. It should be noted that anti-UPA antibody activity was also observed in monkey #5.

### (Summary of the Examples)

(1) By single subcutaneous immunization with the Hub-TGDK-Fetuin vaccine, antibodies could be induced that reacted with both HIV-1 Env protein and SIVmac239 Env protein.
(2) Antibodies reacting with the special conformation (UPA) of the co-receptor CCR5 in HIV-1 were found to have been induced in the anti-serum of monkey #5; quite surprising result was found that the moieties formed by disulfide bonds in Fetuin fitted the UPA of CCR5 so well that Fetuin could induce antibodies against the special conformation of CCR5.
(3) Taken together, by inducing cross immunity in the mucous membrane, mucosal-associated HIV infection can be prevented.
(4) Immunization was first performed with the Hub-TGDK-UPA-CpGODN-gp140-Fetuin vaccine and after anti-gp140 antibodies disappeared from the blood, a booster was given by subcutaneous injection of gp140-free, Hub-TGDK-UPA-CpGODN-Fetuin, whereupon anti-gp140 antibodies could be produced. The similar result was confirmed by boosting with Fetuin alone through subcutaneous injection.
(5) Upon immunization of monkeys with the Hub-TGDK-Fetuin vaccine, the production of antibodies reacting with the gp140 antigen, namely, anti-gp140 antibodies, was confirmed.
(6) If induction of mimicry is established at the site of initial infection by means of an antigen having molecular moieties that mimic the HIV Env glycoprotein from a viewpoint of structural biology, a vaccine can provide complete protection against infection that outperforms the Jenner's smallpox shot.

## Claims

1. An AIDS vaccine comprising a covalent complex of a Hub-antigen, N²,N⁶-bis[N²,N⁶-bis(3,4,5-trihydroxybenzoyl)-L-lysyl]-N-(2-aminoethyl)-L-lysine amide (TGDK), and Fetuin.

2. The AIDS vaccine according to claim 1 which is administered subcutaneously and/or orally and nasally.
